# EUROPEAN PATENT SPECIFICATION

(11) **EP 3 688 233 B1**
(45) Date of publication and mention of the grant of the patent: **05.01.2022**
(21) Application number: 18827272.8
(22) Date of filing: 28.09.2018
(51) Int. Cl.: E02D 1/02, E21B 43/12

(54) **MODULAR DETECTING DEVICE**
MODULARES DETEKTIONSGERÄT
SYSTÈME MODULAIRE DE DETECTION

(30) Priority: 29.09.2017 NL 2019646
(43) Date of publication of application: 05.08.2020
(73) Proprietor: Acacia Water B.V., 2805 BM Gouda (NL)
(72) Inventor: VELSTRA, Jouke, 2805 BM Gouda (NL)
(74) Representative: van Trier, Norbertus Henricus Gerardus
(86) International application number: PCT/NL2018/050643
(87) International publication number: WO 2019/066655

(56) References cited:
- JP-A- H0 988 052
- JP-A- H05 321 235
- US-A- 5 821 405
- US-A1- 2002 148 298
- US-A1- 2017 138 828

## Description

### BACKGROUND

The invention relates to a modular measuring device for measuring at least one parameter of a medium external to, at least partially surrounding and/or inside the modular measuring device, such as an atmospheric, climatic, soil, ground water and/or surface water parameter, and/or for measuring at least one parameter of the modular measuring device itself, such as (relative) inclination, position and/or location. The invention also relates to a processing system for processing one or more measured parameters and/or one or more determined positions received from one or more modular measuring devices. Furthermore, the invention relates to a method for processing one or more measured parameters and/or one or more determined positions received from one or more modular measuring devices.

A measuring pipe, also known as an open or standpipe piezometer, for measuring changes in ground slope and ground water level in soil is known from US 2017/138828 A1 and also from KR 101177459 B. The measuring pipe includes a base pipe, a perforated pipe, a lower finishing tip, a coupling and an upper finishing cap. The perforated pipe is connected to the lower end of the base pipe by means of the coupling. The perforated pipe is formed as a double-walled pipe with an inner perforated pipe and an outer perforated pipe, wherein the inner perforated pipe is inserted into the outer perforated pipe. The inner perforated pipe and the outer perforated pipe are both surrounded by filter paper. The lower finishing tip is connected to the lower end of the perforated pipe and is cone-shaped, such that the lower finishing tip can be inserted easily into a hole in ground. The upper finishing cap covers the upper end of the base pipe. During installation, the measuring pipe is placed into a hole in the ground, after which a layer of sand is provided around the perforated pipe of the measuring pipe. A layer of bentonite is provided on top of the layer of sand and a layer of cement is provided on top of the layer of bentonite. Subsequently, a ground water level meter and an inclinometer are temporarily inserted into the measuring pipe.

### SUMMARY OF THE INVENTION

A disadvantage of the known measuring pipe is that the ground water level or changes in ground slope can only be measured at a predetermined fixed position. Additionally, due to the fixed position of the known measuring pipe, the measuring pipe is placed at the outskirts of a plot of land leading to measurement results being suboptimal or even unsuitable for the objective of the measurement(s) for the user.

It is an object of the present invention to ameliorate or to eliminate one or more disadvantages of the prior art, or to at least provide an alternative measuring device.

According to a first aspect, the invention provides a modular measuring device for measuring at least one parameter of a medium external to, at least partially surrounding and/or inside the modular measuring device, such as an atmospheric, climatic, soil, ground water and/or surface water parameter, and/or for measuring at least one parameter of the modular measuring device itself, such as (relative) inclination, position and/or location, the modular measuring device comprising:
a housing;
a cylindrical module configured to be placed in at least one medium, wherein the cylindrical module is open or closed to the medium and is in data communication with the housing; and
a sensor at least partially arranged within the cylindrical module and configured for measuring at least one parameter of the medium external to, at least partially surrounding and/or inside the cylindrical module, and/or a parameter of the modular measuring device itself,
the modular measuring device further comprising position determining means at least partially arranged within the housing and/or within the cylindrical module, and configured for determining the position of the position determining means, wherein the modular measuring device is configured for transmitting the at least one measured parameter and the determined position to and/or receiving data from an external processing device.

The modular measuring device according to the invention can be used in one or more medium surroundings, such as soil, surface water, ground water and atmosphere. When used in soil, the measuring device or at least the cylindrical module thereof, which can be remote from the housing or can be secured physically to the housing, is inserted in a borehole in the soil, which borehole is drilled by means of, for example, a ground drill. After inserting the modular measuring device into the borehole, the cylindrical module is at least surrounded partially by the soil. The sensor within the cylindrical module then can measure a parameter of and/or in the soil, such as humidity, soil moisture, electrical conductivity, flow, water level, pressure and soil composition and water quality. The measured parameter is subsequently transmitted to the external processing device, for example, through an external and/or an at least partially internal data transmitter and/or transceiver. The positioning determining means can be used to determine the position of the housing and/or the cylindrical module. The determined position is also transmitted to the external processing device, in the same manner as the measured parameter. At the external processing device, e.g. a server, a computer or a mobile device, e.g. a mobile phone, which is in data communication with the modular measuring device through a wired or wireless connection, the received measured parameter and determined position can be combined. The combination of the measured data and determined position(s) gives an accurate representation of the parameter at the specific position. A user of the modular measuring device can place the measuring device at any desired position, after which the position at which the measurement is done is determined by the modular measuring device itself and/or in combination with at least one external and/or internal transceiver and/or processing system. The modular measuring device can therefore be used at any position without the need to determine the respective position in advance, thereby granting the user the freedom to use the modular measuring device at any random position.

It is noted that in the context of the present patent application, position has to be understood as the position, location and/or height in X, Y and/or Z coordinates and/or other positioning measure with respect to a reference and/or inclination relative to a determined reference. Further, determined position also has to be understood as the raw position data which needs to be processed.

In an embodiment, the position determining means is configured for determining the position periodically and/or upon receipt of a request thereto. By determining the position of the modular measuring device periodically or upon receipt of a request thereto, the position determining means can be prevented from determining the position of the modular measuring device continuously. When the position determining means are active for a short period of time, the amount of energy required for operating the position determining means can be kept to a minimum.

In an embodiment, the modular measuring device is configured for determining the position, by means of the position determining means, following the arrangement of the modular measuring device in the at least one medium. The modular measuring device may for example comprise an inclinometer which for example determines when the orientation of the modular measuring device changes, such as from a substantially horizontal orientation to a substantially vertical orientation. An advantage of this embodiment is that the position of the modular measuring device is determined following the placement of the modular measuring device. The external processing device therefore receives the position of the modular measuring device immediately or shortly after the placement.

In an embodiment, the modular measuring device comprises a transmitter, a transceiver and/or receiver arranged at least partially within the housing in data communication with the cylindrical module, the sensor and/or the position determining means, wherein the transmitter and/or transceiver are configured for transmitting the measured parameter and/or the determined position to or from the external processing device. Preferably the transmitter, the transceiver and/or receiver are wired or wirelessly connected to the cylindrical module, the sensor and/or the position determining means. An advantage of this embodiment is that the measured parameter and the determined position can be, for example, received remotely through a processing system. When a transceiver is provided, the modular measuring device can receive instructions pertaining to measuring the parameter and/or determining the position. Further, it can also be possible to transmit for example calibration or configuration data to the modular measuring device. The calibration or configuration data can be used for calibrating or configuring the modular measuring device.

In an embodiment, the transmitter, the transceiver and/or the receiver are configured to operate in a wired and/or mobile network, such as a Long Range Wide Area Network (LoRaWAN^{™}), a 2G network, a 3G network, a 4G network and/or any other mobile network.

In an embodiment, the modular measuring device comprises an additional transmitter, an additional transceiver, and/or an additional receiver arranged at least partially within the housing and in data communication with the cylindrical module, sensor and/or the position determining means for transmitting the measured parameter and the determined position to and/or from the external processing device. Preferably the transmitter, the transceiver, and/or the receiver are configured to operate in one of the Long Rang Wide Area Network (LoRaWAN^{™}), the 2G network, the 3G network, the 4G network and/or any other mobile network, and the additional transmitter, the additional transceiver, and/or the additional receiver are configured to operate in another one of the Long Range Wide Area Network (LoRaWAN^{™}), the 2G network, the 3G network, the 4G network and/or any other mobile network. For example, when the transceiver is configured to operate in the LoRaWAN^{™} and the additional transceiver is configured in the 4G network, small data packets can be transmitted or received through the transceiver and large data packets can be transmitted or received through the additional transceiver.

In an embodiment, the cylindrical module comprises a module transmitter, a module transceiver, and/or a module receiver, which module transmitter, module transceiver, and/or module receiver are in data communication with the housing, preferably the housing and the cylindrical module comprise position determining means. An advantage of this embodiment is that the cylindrical module can be placed at a distance from the housing and/or a module wireless or wired connected to the housing.

In an embodiment, the housing comprises a first side and a second side opposite to the first side, and the cylindrical module has opposite ends, wherein the cylindrical module is detachably secured to the first side of the housing at one of the opposite ends thereof. In a further embodiment, an additional cylindrical module is provided between the housing and the cylindrical module, preferably wherein the additional cylindrical module is a spacing cylindrical module. An advantage of this embodiment is that the sensor within the cylindrical module can be placed at any given distance from the housing by providing the additional cylindrical module between the housing and the cylindrical module.

In an embodiment, the modular measuring device comprises one or more additional cylindrical modules with opposite ends, which are in data communication with the cylindrical module, another additional cylindrical module and/or the housing. Optionally, the one or more additional cylindrical modules comprise position determining means for determining the position of the respective additional cylindrical module.

In an embodiment, the one or more additional cylindrical modules are detachably secured in series to the cylindrical module, to another one of the one or more additional cylindrical modules, and/or to the second side of the housing at one of the opposite ends thereof. In a further embodiment, each of the one or more additional cylindrical modules is a spacing cylindrical module and/or is provided with an additional sensor configured for measuring a parameter of the medium external to, surrounding and/or inside the respective cylindrical module, or for measuring a parameter of the modular measuring device itself. By providing one or more spacing modules, it is possible to locate the sensor for example at any given distance from the housing. By providing one or more additional cylindrical modules provided with a sensor, it is possible to measure two or more parameters with one modular measuring device. When an additional cylindrical module with a sensor is secured to the second side of the housing, it is for example possible to measure a parameter of the soil, in which at least the cylindrical module is placed, and a parameter of for example air above the soil, in which the additional cylindrical module is placed. The additional cylindrical module(s) with a sensor or the spacing module(s) can be easily replaced or removed.

In an embodiment, the housing, the cylindrical module and/or the one or more additional cylindrical modules comprises a memory containing identification data related to the housing or the respective cylindrical module, which memory is in data communication with the housing. Preferably the identification data relates to whether the additional cylindrical module is a spacing module or a cylindrical module with a sensor, to the parameter measured by the sensor, the sensor, the position determining means, the sensor, the position determining means, and/or to the length of the respective cylindrical module. In an embodiment, the modular measuring device is configured to transmit and/or to confirm the identification data of each of the housing, the cylindrical module and/or the one or more additional cylindrical modules to the external processing device, preferably upon a request thereto. By transmitting the identification data to the external processing device, it is for example possible to determine remotely which sensors are available in the modular measuring device. It is therefore possible to establish remotely which sensors are used. Additionally, it is possible to determine the position(s) of the cylindrical module and the one or more additional cylindrical modules and/or sensor(s) in the modular measuring device and/or external to the modular measuring device.

In an embodiment, the modular measuring device is configured to transmit the identification data of the housing, the cylindrical module and/or the one or more additional cylindrical modules in a transmitting order, and the cylindrical module and the one or more additional cylindrical modules are arranged in a module order, wherein the transmitting order corresponds to or is inverse to the module order. By means of the identification data, it is possible, among others, to determine the distance between the housing and one of the sensors and/or position determining means. Since the distance between the housing and the sensor can be determined, the position of the respective sensor can also be determined. The measured parameter can be related to for example a national reference value for the measured parameter, such as Normal Amsterdam Level (N.A.P) for the ground water level.

In an embodiment, each of the cylindrical module and the one or more additional cylindrical modules has a first coupling part at one of the opposite ends and a second coupling part at the other of the opposite ends. Preferably, the first coupling part of each of the cylindrical module and the one or more additional cylindrical modules can be secured with the second coupling part of an adjacent cylindrical module, or vice versa.

In an embodiment, each of the cylindrical module and the one or more additional cylindrical modules has a first connector at one of the opposite ends, a second connector at the other of the opposite ends, and one or more conductors for conducting energy and/or data between the first connector and the second connector, or vice versa. In a further embodiment, for each of the cylindrical module and the one or more additional cylindrical modules, the first connector is provided at or near the first coupling part and the second connector is provided at or near the second coupling part. An advantage of this embodiment is that by securing the cylindrical modules to each other, a data path and/or an energy path can be established between the cylindrical modules, thereby enabling communication between for example a sensor in an additional cylindrical module and the transceiver arranged within the housing.

In an embodiment, the position determining means is selected from a group comprising a Global Positioning System (GPS) receiver and a Global Navigation Satellite System (GNSS) receiver.

In an embodiment, an additional sensor is provided at least partially within or connected to the housing and/or the cylindrical module. It is thereby possible to measure an additional parameter of a medium surrounding and/or inside the housing of the modular measuring device. A rain sensor can, for example, be connected to the housing, such that it is possible to determine the amount of rain at a position where the modular measuring device is placed.

In an embodiment, the sensor or the additional sensor is selected from a group comprising a humidity sensor, a flow sensor, a pH sensor, a temperature sensor and an (in)clinometer.

In an embodiment, the modular measuring device comprises a storage medium arranged within the housing, wherein the storage medium is configured for storing the one or more measured parameters and the determined position at least temporarily. During use, it may occur that the modular measuring device, for example, cannot reach the external processing device. By storing the one or more measured parameters and the determined position at the storage temporarily, the measured parameters and the determined position can be saved. The saved measured parameters and determined position, optionally, can be transmitted to the external processing device at a later time.

In an embodiment, the modular measuring device, in particular the housing, comprises a connector configured for connecting to an external device, wherein the connector is in data communication with the housing and/or the cylindrical module. The external device, for example, can be a laptop or a mobile phone, which can be connected to connector of the housing wireless or wired. An advantage of this embodiment is that data can be downloaded from the modular measuring device to the external device, or vice versa, via the connector.

In an embodiment, the modular measuring device comprises signaling means arranged at least partially within the housing, wherein the signaling means are configured for emitting a signal, wherein the emitted signal is at least one of an alarm signal, a recognition signal and a signaling signal. In a further embodiment, the signaling means comprise at least one of a Bluetooth transmitter, a light source, such as an infrared light or an LED, and a sound source for emitting the signal. The signal can be emitted continuously, periodically or when triggered thereto. The emitted signal can be received by, for example, a mobile phone of the user, such that the user is made aware of the presence of the modular measuring device. It can therewith be prevented that the user drives over the modular measuring device, since the user is warned in advance.

Furthermore, the emitted signal can provide an indication of the modular measuring device itself. The indication, for example, can relate to whether the modular measuring device is working properly or to whether maintenance is needed.

According to a second aspect, the invention provides a processing system for processing one or more measured parameters and/or one or more determined positions from one or more modular measuring devices, the processing system comprising one or more modular measuring devices according to the first aspect of the invention, the processing system further comprising:
a processing device in data communication with the one or more modular measuring devices for receiving one or more measured parameters and/or one or more determined positions from the one or more modular measuring devices, wherein the processing device is configured for processing the one or more measured parameters and the one or more determined positions received from each of the one or more modular measuring devices.

An advantage of this processing system is that the one or more modular measuring devices can be placed at any desired position, which position can be determined by the processing system after placement of the modular measuring device.

A further advantage is that the measured parameters and the determined positions of all modular measuring devices can be processed at any at least one location. A user therefore has access to all measured parameters and determined positions from any location with access to data processing and/or displaying devices and/or means.

Furthermore, since the processing device processes the one or more measured parameters, one or more determined positions data and/or identification data received from each of the one or more modular measuring devices, computing power can be provided at the processing device while no sophisticated electronics are required at the modular measuring devices. By processing the identification data from the one or more modular measuring devices, it is possible to determine the configuration of each of the one or more modular measuring devices.

In an embodiment, the processing device further comprising a data storage configured for storing the one or more measured parameters, one or more determined positions, and/or any data received from and/or sent to the one or more modular measuring devices.

In an embodiment, the processing device is configured to transmit a command to at least one of the one or more modular measuring devices in order to measure the one or more parameters and/or to determine the positions of the one or more modular measuring devices.

In an embodiment, during use, the processing device receives a plurality of determined positions from each of the one or more modular measuring devices, wherein the processing device is configured, for each of the one or more modular measuring devices, to determine an actual position based on the plurality of determined positions. An advantage of this embodiment is that by determining the actual position from a plurality of determined positions of each of the modular measuring devices, the accuracy of determining the actual position of the modular measuring device can continuously be improved. In order to determine the actual position accurately, Precise Point Positioning can be applied, for example.

In an embodiment, the one or more modular measuring devices comprises one or more additional cylindrical modules, wherein each of the housing, the cylindrical module and/or the one or more additional cylindrical modules comprises a memory containing identification data related to the housing or the respective cylindrical module, which memory is in data communication with the housing. Preferably the identification data relates to whether the additional cylindrical module is a spacing module or a cylindrical module with a sensor, to the parameter measured by the sensor, the sensor, the position determining means, and/or to the length of the respective cylindrical module. The modular measuring device is configured to transmit and/or to confirm the identification data of each of the housing, the cylindrical module and/or the one or more additional cylindrical modules to the external processing device, preferably upon a request thereto. Preferably the modular measuring device is configured to transmit the identification data of each of the housing, the cylindrical module and/or the one or more additional cylindrical modules in a transmitting order, and the cylindrical module and the one or more additional cylindrical modules are arranged in a module order, wherein the transmitting order corresponds to or is inverse to the module order. The processing device is configured to transmit an identification command to at least one of the one or more modular measuring devices, to receive identification data of the housing, the cylindrical module and/or the one or more additional cylindrical modules from at least one of the one or more modular measuring devices, and to determine the module order in which the cylindrical module and the one or more additional cylindrical modules are arranged for each of the one or more modular measuring devices. Since the position of the housing of a modular measuring device is determined, as is the distance between a sensor and the housing, it is possible to determine the coordinates at which a sensor has performed the measurement. This embodiment has as the advantage that the measured parameters and/or data of the modular measuring device can be shown in or incorporated into commercially available maps.

In an embodiment, the processing system comprises a forecasting, nowcasting and/or backcasting device configured for forecasting, nowcasting and/or backcasting one or more parameters of the medium surrounding at least a part of one of the one or more modular measuring devices, preferably wherein the forecasting, nowcasting and/or backcasting device uses at least one computer model for forecasting, nowcasting and/or backcasting the one or more parameters and/or other data. In a further embodiment the processing system is configured to adjust the at least one computer model on basis of the one or more measured parameters. An advantage of this embodiment is that measurements performed by the one or more modular measuring devices can be used for improving an existing computer model, such that the predictions of the computer model can be validated, calibrated and optimized.

According to a third aspect, the invention provides a method for processing one or more measured parameters and/or one or more determined positions received from one or more modular measuring devices according to the first aspect of the invention, by means of a processing system according to the second aspect of the invention, the method comprising the steps of:
for each of the one or more modular measuring devices, positioning the modular measuring device at any desirable position in which the modular measuring device is at least partially surrounded by a medium,
for each of the one or more modular measuring devices, measuring, with the use of the modular measuring device, one or more parameters of the medium external to, at least partially surrounding and/or inside the modular measuring device, and/or one or more parameters of the modular measuring device itself,
for each of the one or more modular measuring devices, determining, with the use of the modular measuring device, the position of the modular measuring device, and
for each of the one or more modular measuring devices, transmitting the measured one or more parameters and/or determined position to the processing device.

Optionally, the method comprises the step of providing one or more modular measuring devices according to the first aspect of the invention, and/or a processing system according to the second aspect of the invention.

In an embodiment, the method comprises the steps of:
for each of the one or more modular measuring devices, forecasting, nowcasting and/or backcasting at least one parameter of the medium surrounding and/or inside the respective modular measuring device at least partially, by using a computer model,
comparing the forecasted, nowcasted and/or backcasted parameter with a respective one of the measured parameters, and
providing feedback to and adjusting the computer model based on a difference between the forecasted, nowcasted and/or backcasted parameter and the respective one of the measured parameters.

In an embodiment, the modular measuring device comprises one or more additional cylindrical modules with opposite ends, which are detachably secured in series to the cylindrical module, wherein each of the housing, the cylindrical module and/or the one or more additional cylindrical modules comprises a memory containing identification data related to the housing or the respective cylindrical module, which memory is in data communication with the housing, wherein the cylindrical module and the one or more additional cylindrical modules are arranged in a module order, and wherein the method comprises the steps of
transmitting an identification command from the processing device to at least one of the one or more modular measuring devices,
at the at least one of the one or more modular measuring devices, transmitting, upon receipt of the identification command, the identification data of each of the housing, the cylindrical module and/or the one or more additional cylindrical modules to the processing device, and
at the processing device, processing the identification data received from the at least one modular measuring device,
wherein the identification data of the housing, the cylindrical module and/or the one or more additional cylindrical modules are transmitted in a transmitting order, wherein the transmitting order corresponds to or is inverse to the module order.

The various aspects and features described and shown in the specification can be applied, individually, wherever possible. These individual aspects, in particular the aspects and features described in the attached dependent claims, can be made subject of divisional patent applications.

### BRIEF DESCRIPTION OF THE DRAWINGS

The invention will be elucidated on the basis of exemplary embodiments shown in the attached drawings, in which:
Figure 1A shows an isometric view of an embodiment of a modular measuring device according to the invention with a housing and a first, second, third, fourth and fifth cylindrical module;
Figure 1B shows an exploded view of the modular measuring device of figure 1A;
Figure 2A shows an isometric view of the housing and first cylindrical module of figures 1A;
Figure 2B shows a cross-section view of the first cylindrical module of figure 2A according to plane IIB;
Figure 3A shows an isometric view of the second cylindrical module;
Figure 3B shows an isometric view of the third cylindrical module;
Figure 3C shows a cross-section view of the third cylindrical module of figure 3B according to plane IIIC;
Figure 3D shows an isometric bottom view of the third cylindrical module of figure 3B; and
Figure 4 shows a schematic overview of a processing system for processing data from the one or more modular measuring devices of figure 1A according to an embodiment of the invention.

### DETAILED DESCRIPTION OF THE INVENTION

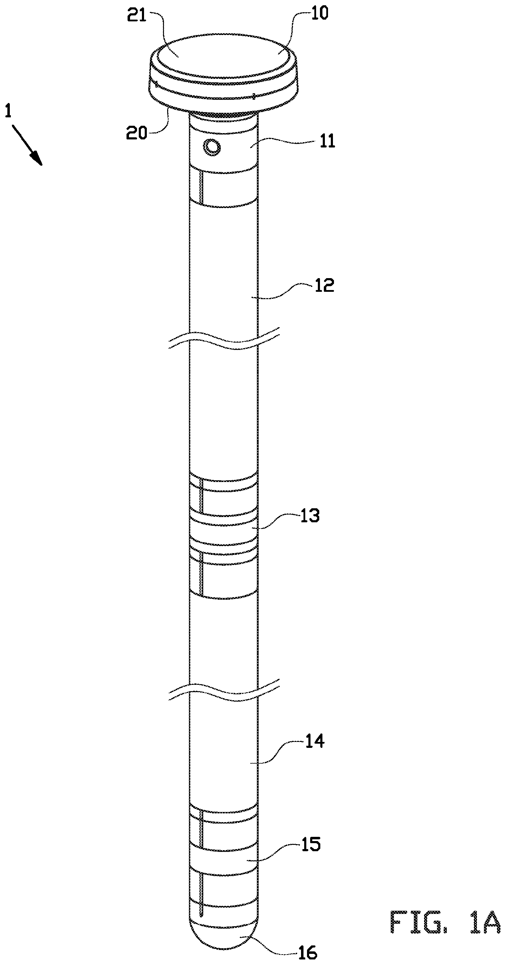

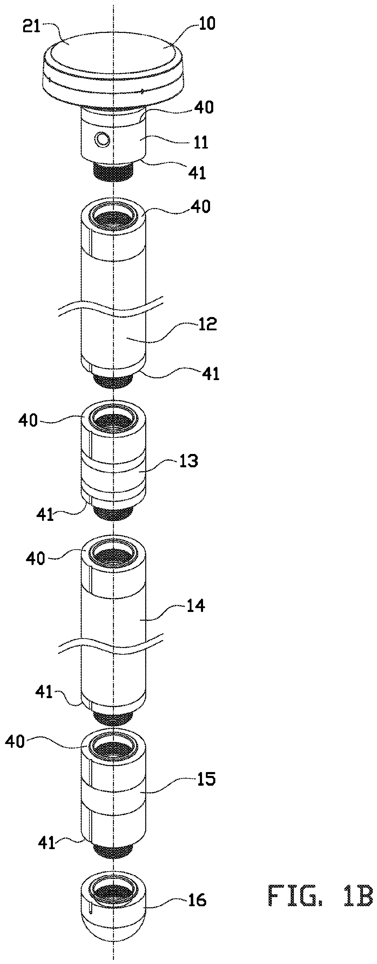

An isometric view of the modular measuring device 1 according to an embodiment of the invention is shown in figure 1A. The measuring device 1 comprises a housing 10, a first cylindrical module 11, a second cylindrical module 12, a third cylindrical module 13, a fourth cylindrical module 14 and a fifth cylindrical module 15. The housing 10 has a first side 20 and a second side 21. As shown in figure 1B each of the first cylindrical module 11, the second cylindrical module 12, the third cylindrical module 13, the fourth cylindrical module 14 and the fifth cylindrical module 15 has a first end 40 and a second end 41 as shown in figure 1B. Each second end 41 of each of the cylindrical modules 11, 12, 13, 14, 15 is detachably secured to the first end 40 of the respective adjacent cylindrical module 11, 12, 13, 14, 15, in this drawing the cylindrical module placed beneath it. The series of cylindrical modules 11, 12, 13, 14, 14 is secured to the first side 20 of the housing 10. A stop 16 is secured to the second end 41 of the fifth module 15.

The modular measuring device 1 is intended to be placed in a surrounding medium, e.g. soil, air, an atmosphere, ground water or surface water, wherein the soil, air or surface water surrounds the cylindrical modules 11, 12, 13, 14, 15 and the stop 16, and/or surrounds or connects to a sensor in one of the cylindrical modules 11, 12, 13, 14, 15. The first, third and fifth cylindrical modules, 11, 13, 15 are provided with a sensor which is arranged at least partially within the respective cylindrical modules 11, 13, 15, as is explained in more detail below. Parameters of the soil or surface water, such as electrical conductivity, humidity and temperature, can be measured by means of the sensors within the respective cylindrical modules 11, 13, 15. It is also possible to determine a parameter of the modular measuring device 1 itself, such as acceleration and inclination. The second and fourth cylindrical modules 12, 14 are spacing cylindrical modules, which are provided in order to be able to place the sensor(s) at a predetermined distance from the housing 10. It is noted that each of the cylindrical modules 11, 12, 13, 14, 15 can be provided with a sensor or can be used as a spacing module; as such the modular measuring device 1 can be adapted to the wishes of a user.

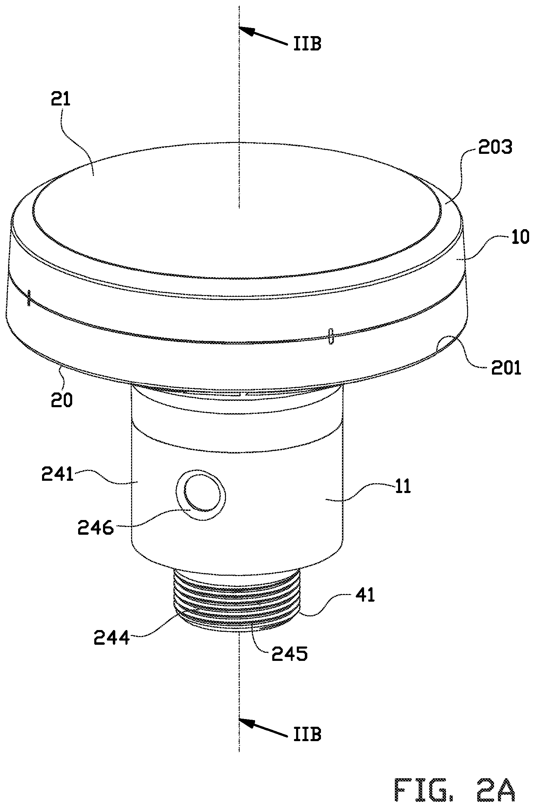

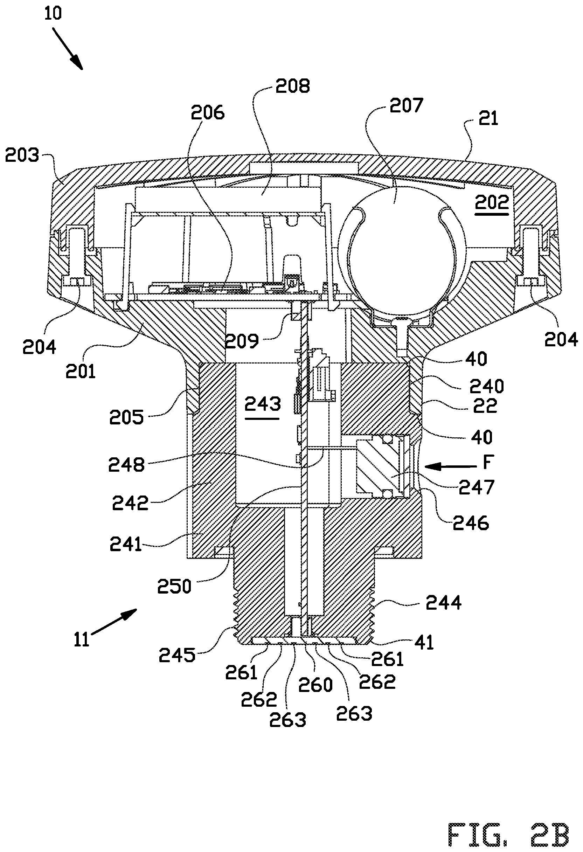

The housing 10 and the first cylindrical module 11 are shown in more detail in figures 2A and 2B. The housing 10 comprises a bottom part 201 and a top part 203, whereby a space 202 is defined by the bottom part 201 and the top part 203. The bottom part 201 and the top part 203 are connected to each other by means of connecting elements 204, such as screws. As shown in figure 2B the bottom part 201 has a coupling opening 205 which is configured for receiving a first coupling part 240 of the first cylindrical module 11 at the first end 40 thereof. The housing 10 further comprises a main circuit board 206, a battery 207 and an electronic unit 208 with positioning determining means and a transceiver. The main circuit board 206 is electrically connected to the battery 207 and to the electronic unit 208, such that the battery 207 provides electrical energy to the main circuit board 206 and the electronic unit 208. The main circuit board 206 further comprises a connecting member 209 for connecting to a module circuit board 250 provided within the first cylindrical module 11 which is secured to the housing 10.

The housing 10 may further comprises a storage medium (not shown) for storing the one or more measured parameters and/or the determined position, as well as signaling means configured for emitting a signal, wherein the emitted signal is at least one of an alarm signal, a recognition signal and a signaling signal.

The position determining means of the electronic unit 208 are configured for determining the position of the modular measuring device 1 for example by means of a non-shown Global Positioning System (GPS) or Global Navigation Satellite System (GNSS). The position determining means are configured to determine the position of the measuring device 1 once, periodically, or upon receipt of a command thereto via the transceiver within the electronic unit 208. Each time the position of the measuring device 1 has been determined, the determined position is transmitted to an external processing device (not shown) by the transceiver. When the position of the measuring device 1 is determined periodically, the external processing device can combine a plurality of determined positions into an actual position in order to determine the actual position of the measuring device 1 very accurate, i.e. with an accuracy of centimeters.

It is noted that since the measuring device 1 is able to determine and transmit its position, the measuring device 1 can be placed at any desired position. During use of the measuring device 1, the position is determined, such that the user knows at which position the measurements by the measuring device 1 have been performed. The modular measuring device 1 is therefore a mobile modular measuring device 1.

As shown in figure 2B the first cylindrical module 10 comprises a partially hollow module body 241 with a wall 242 that defines a space 243 within the module body 241. The module body 241 comprises the first coupling part 240 at the first end thereof and comprises a second coupling part 244 at the second end 41 thereof. As shown in figures 2A and 2B the second coupling part 244 has an external thread 245. An aperture 246 is provided within the wall 242 of the module body 241, which aperture 246 opens into the space 243 of the module body 241. A sensor 247 is arranged within the space 243, such that it blocks the aperture 246 in the wall 242. A force F exerted by the surrounding medium is therefore applied to the sensor 247. As shown in figure 2B, when the first cylindrical module 11 is secured to the housing 10, the module circuit board 250 is placed within the space 243 and is connected to the connecting member 209 of the main circuit board 206, such that data and/or electrical energy can be transmitted from the main circuit board 206 to the module circuit board 250, or vice versa. The sensor 247 is in data and energy communication with the module main circuit board 206 via a conduit 248. In use, the sensor 247 is powered via the main circuit board 206, the module circuit board 250 and the conduit 248, and measurement data from the sensor 247 can be transmitted to the main circuit board 206.

As shown in figure 2B the module circuit board extends over substantially the complete height of the space 243 of the first cylindrical module 11. At the second end 41 of the first cylindrical module 11 and in communication with the module circuit board 250, the cylindrical module 11 has a lower connector 260 with lower connector elements 261, 262, 263, 264 for communicating with the second cylindrical module 12 that is connected to the second end 41 of the first cylindrical module 11, such that it is possible to transmit energy and data between the first and cylindrical module 11, 12, which energy and data is transmitted from or towards the main circuit board 206. The lower connector 260 with lower connector elements 261, 262, 263, 264 is explained in more detail below.

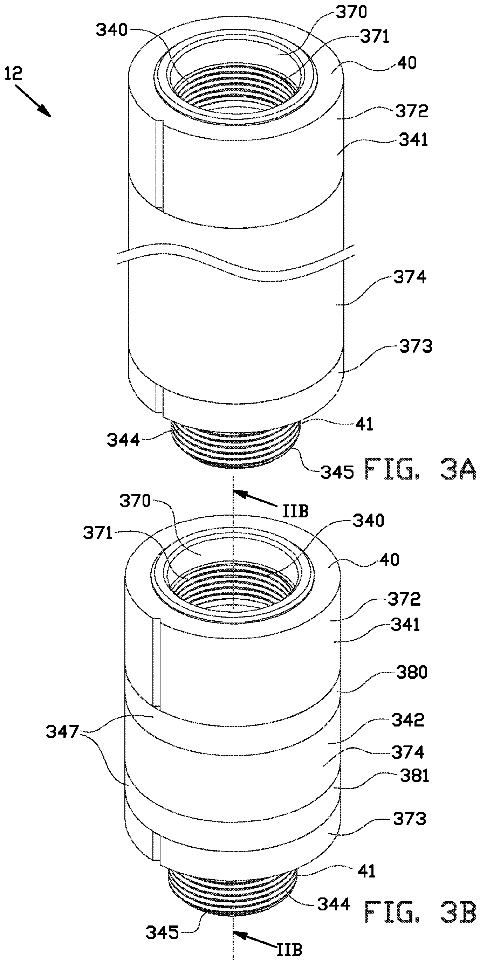

An isometric view of the second cylindrical module 12 and the third cylindrical module 13, respectively, is shown in figures 3A and 3B. The second and third cylindrical modules correspond largely to each other and therefore the corresponding features are described at once.

Each of the second and third cylindrical modules 12, 13 comprises a partially hollow module body 341 with a space 343 therein and having a first coupling part 340 at the first end 40 thereof. The first coupling part 340 is formed by a cylindrical recess 370 in the partially hollow module body 341. As shown in figures 3A and 3B an inner wall of the cylindrical recess 370 is provided with an internal thread 371. Each of the second and third second cylindrical modules 12, 13 also has a second coupling part 344 at the second end 41 thereof. As shown in figures 3A and 3B the second coupling part 344 has an external thread 345. When for example the third cylindrical module 13 is secured to the second cylindrical module 12, the second coupling part 344 of the second cylindrical module 12 is inserted into the cylindrical recess 370 of the first coupling part 340 of the third cylindrical module 13. As a consequence, the internal thread 371 and the external thread 345 are brought in contact with each other, and the second and third cylindrical modules 12, 13 can be secured to each other by rotating one of the second and third cylindrical module 12, 13 about the longitudinal axis thereof.

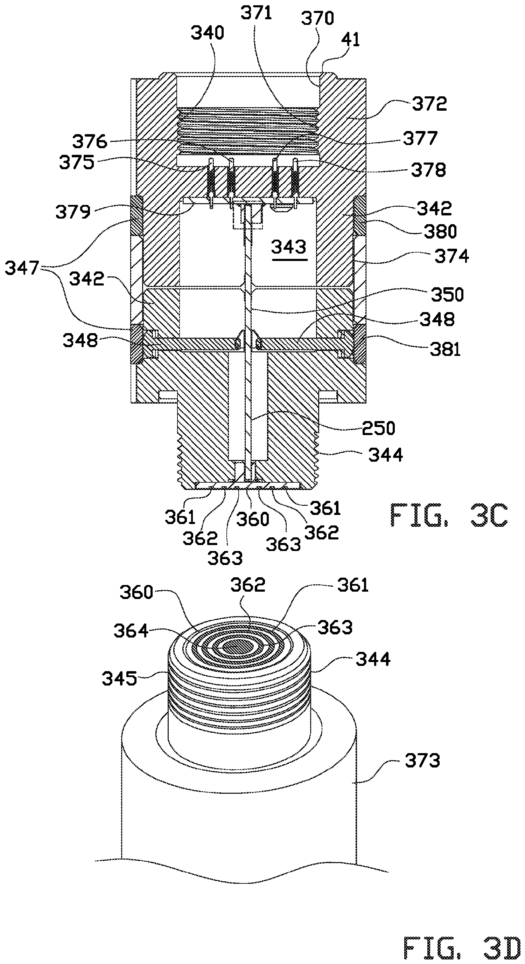

As shown in figure 3C the partially hollow body 341 has an upper body part 372 and a lower body part 373 which are placed on top of each other and held together by means of a casing 374 which encloses a part of both of the upper body part 372 and the lower body part 373. The upper and lower body part 373 can be separated from each other, such that a user has access to the space 343 within the module body 341.

As shown in figure 3C a module circuit board 350 is placed within the space 343 of the partially hollow body 341, which module circuit board 350 extends over substantially the complete height of the space 343 of the cylindrical module 12, 13. At the second end 41 of the cylindrical module 12, 13 and in communication with the lower side of the module circuit board 350, the cylindrical module 12, 13 has a lower connector 360 with four lower connector elements 361, 362, 363, 364 for communicating with a cylindrical module secured thereto. As shown in figure 3D each of the lower connector elements 361, 362, 363, 364 is circular shaped and separated from the other connector elements. As shown in figure 3C the cylindrical module 12, 13 is provided with an upper connector 379 which is in communication with the upper side of the module circuit board 350. The upper connector 379 has four upper connector elements 375, 376, 377, 378 extending upwards therefrom and into the recess 370. When for example the second cylindrical module 12 is secured to the third cylindrical module 13, the upper connector elements 375, 376, 377, 378 of the third cylindrical module 13 are respectively in contact with the four lower connector elements 361, 362, 363, 364 of the second cylindrical module 12. By providing separate connector elements 361, 362, 363, 364, 375, 376, 377, 378, separate data and energy paths can be provided throughout the modular measuring device 1.

It is noted that the fourth and fifth cylindrical module 14, 15 comprise substantially the same features. As schematically indicated in figure 3A the second cylindrical module 12 can have any desired length in order to determine the between the housing 10 and the third, fourth and fifth cylindrical modules 13, 14, 15 respectively. This is also applicable to the fourth cylindrical module 14.

The third cylindrical module 13 differs from the second cylindrical module 12 in that it comprises a sensor 347. The sensor 347 comprises a first metal ring 380 and a second metal ring 381 which are provided at the outside of the partially hollow body 341 and above each other. As shown in figure 3C the second metal ring 381 is in communication with the module circuit board 350 via a conduit 348. The first metal ring 380 is also in communication with the module circuit board 350 by a non-shown conduit. During use, an electrical circuit is formed with among others the module circuit board 350, the first metal ring 380 and the second metal ring 381, in order to determine the electrical conductivity of the medium surrounding at least the third cylindrical module 13. The sensor 347 is powered by the battery 207 in the housing 10, and measurement data is transmitted from the sensor 347 to the transceiver within the housing via the intermediate module circuit board(s) 350, the connector elements 361, 362, 363, 364, 375, 376, 377, 378 and the main circuit board 206.

It is noted that the module circuit board 350 of each of the cylindrical modules 11, 12, 13, 14, 15 is provided with a memory (not shown) comprising identification data related to the respective cylindrical module 11, 12, 13, 14, 15. The identification data relates to for example whether the respective cylindrical module 11, 12, 13, 14, 15 is a spacing module, such as the second and fourth cylindrical module 12, 14, or a cylindrical module with a sensor, such as the first, third and fifth cylindrical module 11, 13, 15, to the parameter measured by the sensor, and to the length of the respective cylindrical module 11, 12, 13, 14, 15. When the modular measuring device 1 is placed in a medium, the external processing device (not shown) can send an identification command to the transceiver of the measuring device 1. Upon receipt of the identification command, each of the cylindrical modules 11, 12, 13, 14, 15 is instructed to transmit its identification data to the transceiver which transmits the received identification data to the external processing device. The identification data of the cylindrical modules 11, 12, 13, 14, 15 is received by the external processing device in the order in which the cylindrical modules 11, 12, 13, 14, 15 are secured to the housing 10. Thus, in the case of the modular measuring device 1 as shown in figure 1A, the external processing device first receives the identification data of the first cylindrical module 11, thereafter of the second cylindrical module 12, and so on. Since the order of the cylindrical modules 11, 12, 13, 14, 15 is known, as is the length of each of the cylindrical modules 11, 12, 13, 14, 15 and the global position of the measuring device 1, it is possible to determine for example the coordinates at which a measurement is done by a sensor.

Although it is not shown, it is noted that in another embodiment data transmission between the housing 10 and each of the cylindrical modules 11, 12, 13, 14, 15 can be performed wirelessly. Optionally, the module circuit board 350 of each of the cylindrical modules 11, 12, 13, 14, 15 is provided with position determining means (not shown).

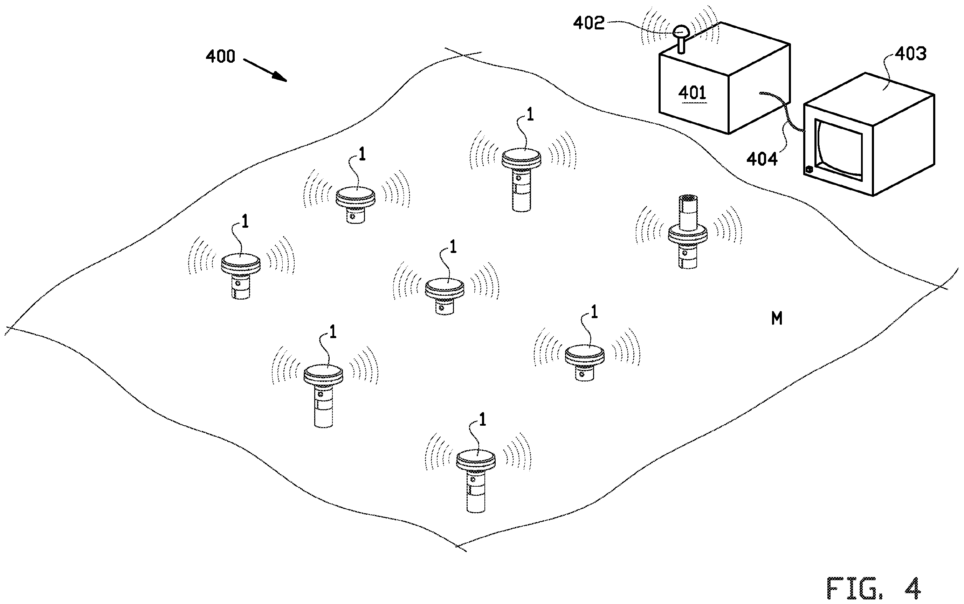

A schematic overview of a processing system for processing data from one or more of the modular measuring devices 1 is shown in figure 4. The modular measuring devices 1 of figure 4 correspond to the modular measuring device 1 as shown in figures 1A and 1B. The processing system 400 comprises a plurality of the modular measuring devices 1. As shown in figure 4 the modular measuring devices 1 are placed in a medium M, in this example soil. The processing system 400 further comprises the external processing device 401, in this case a central server 401, with a transceiver 402. The central server 401 is in data communication with each of the measuring devices 1 placed in the soil M. Each of the measuring devices 1 comprises one or more sensors for measuring one or more parameters of the soil M that surrounds each of the measuring devices 1, or for measuring one or more parameters of the measuring devices 1 itself. The one or more parameters of the soil are for example, but not limited to, humidity, ground water level, ground water flow, and nitrate level in ground water. The parameters of a modular measuring device 1 are for example, but not limited to, acceleration and inclination.

During use each of the measuring devices 1 measures one or more parameters and the measured parameters are subsequently transmitted to the central server 401 via the transceiver of the respective measuring device 1 and the transceiver 402 of the central server 401. It is noted that the measuring devices 1 measure the parameters periodically or upon receipt of a command thereto from the central server 401. Simultaneously with the measuring of the one or more parameters, it is possible to determine the position of each of the measuring devices 1 by means of the global positioning means of the respective measuring device 1, which determined global position is transmitted simultaneously with the measured one or more parameters. Upon receipt of the measured parameters and the position from each of the modular measuring devices 1, the central server 401 processes the measured parameters and the position. For example, the central server 401 maps the measured parameters in combination with the positions, resulting in a map with the measured parameters shown therein. Such a map can for example be used to provide to the user an indication of a ground water level distribution. The central server 401 is provided with a data storage (not shown) for storing the received measured parameters and the determined positions of the modular measuring devices 1.

The processing system 400 further comprises a displaying device 403 which is connected to the central server 401 by means of a data line 404. The mapped measured parameters in combination with the positions can be transmitted from the central server 401 to the displaying device 403, such as a computer, a mobile device or a mobile phone, via the data line 404. A user can observe the measured parameters with the determined positions in map form, but is also possible that the measured parameters with the determined position are shown in a list, for example.

The position of each of the measuring devices 1 can be determined and transmitted once or multiple times. When the position of each of the measuring devices 1 is determined and transmitted multiple times, the central server 401 uses and combines all or a majority of the plurality of determined positions to determine an actual position. Said actual position can reduce errors in the determined position. As a result, the global position of each of the measuring devices 1 can be measured more accurately. Due to the accurately determined global position, the measured parameters can be related to for example a national reference value for the measured parameter, such as Normal Amsterdam Level (N.A.P) for ground water level.

A computer model, for example a geohydrological model, can be used for predicting the expected level of a parameter of a medium, such as ground water level. Such a computer model predicts the expected level of the parameter of the medium within a range or with a predetermined error margin. The central 401 is configured for comparing the predicted parameter with the corresponding measured parameter, in order to determine the difference between the predicted parameter and the corresponding measured parameter. The determined difference is used as feedback for the used computer model such that the computer model can be adapted, whereby the accuracy of the computer model improves by means of the feedback.

It is noted that the measured parameters of the modular measuring device itself, such as movement and inclination, can be used for determining whether the modular measuring device is still in its intended position or has been removed.

It is also possible to provide a modular measuring device for measuring at least one parameter of a medium external to, at least partially surrounding and/or inside the modular measuring device, such as an atmospheric parameter or ground water level, and/or for measuring at least one parameter of the modular measuring device itself, such as inclination, the measuring device comprising: a housing having a first side and a second side opposite to the first side; one or more cylindrical modules having opposite ends and which are detachably secured in series to the first side of the housing, wherein the cylindrical module are configured to be placed in a medium; and a sensor arranged at least partially within at least one of the cylindrical modules and which is configured for measuring a parameter of the medium external to, surrounding and/or inside the cylindrical module and/or for measuring a parameter of the modular measuring device itself, wherein the housing is configured for transmitting the measured parameter to an external processing device, wherein each of the cylindrical modules comprises a memory containing identification data related to the housing or the respective cylindrical module, which memory is in data communication with the housing, wherein each of the cylindrical modules is configured to transmit its identification data to the housing, preferably upon a request thereto, wherein the modular measuring device transmits the received identification data to the external processing device, wherein the identification data of each of the cylindrical modules is transmitted in a transmitting order and the cylindrical modules are arranged in a module order, wherein the transmitting order corresponds to the module order.

## Claims

1. Modular measuring device (1) for measuring at least one parameter of a medium external to, at least partially surrounding and/or inside the modular measuring device, such as an atmospheric, climatic, soil, ground water and/or surface water parameter, and/or for measuring at least one parameter of the modular measuring device itself, such as (relative) inclination, position and/or location, the modular measuring device comprising:
a housing (10); and
a cylindrical module (11, 12, 13, 14, 15) configured to be placed in at least one medium, wherein the cylindrical module is open or closed to the medium and is in data communication with the housing; **characterized by**
a sensor (247, 347) at least partially arranged within the cylindrical module and configured for measuring at least one parameter of the medium external to, at least partially surrounding and/or inside the cylindrical module, and/or a parameter of the modular measuring device itself,
the modular measuring device further comprising position determining means at least partially arranged within the housing and/or within the cylindrical module, and configured for determining the position of the position determining means, wherein the modular measuring device is configured for transmitting the at least one measured parameter and the determined position to and/or receiving data from an external processing device (401).

2. Modular measuring device (1) according to claim 1, wherein the position determining means is configured for determining the position periodically and/or upon receipt of a request thereto,
and/or wherein the modular measuring device is configured for determining the position, by means of the position determining means, following the arrangement of the modular measuring device in the at least one medium,
and/or comprising a transmitter, a transceiver and/or receiver arranged at least partially within the housing (10) in data communication with the cylindrical module (11, 12, 13, 14, 15), the sensor (247, 347) and/or the position determining means, wherein the transmitter and/or transceiver are configured for transmitting the measured parameter and/or the determined position to or from the external processing device (401), preferably wherein the transmitter, the transceiver and/or receiver are wired or wirelessly connected to the cylindrical module (11, 12, 13, 14, 15), the sensor (247, 347) and/or the position determining means,
preferably wherein the transmitter, the transceiver and/or the receiver are configured to operate in in a wired and/or mobile network, such as a Long Rang Wide Area Network (LoRaWAN^{™}), a 2G network, a 3G network, a 4G network and/or any other mobile network,
preferably comprising an additional transmitter, an additional transceiver, and/or an additional receiver arranged at least partially within the housing (10) and in data communication with the cylindrical module (11, 12, 13, 14, 15), sensor (247, 347) and/or the position determining means for transmitting the measured parameter and the determined position to and/or from the external processing device (401), preferably wherein the transmitter, the transceiver, and/or the receiver are configured to operate in one of the Long Range Wide Area Network (LoRaWAN^{™}), the 2G network, the 3G network, the 4G network and/or any other mobile network, and the additional transmitter, the additional transceiver, and/or additional recveiver are configured to operate in another one of the Long Range Wide Area Network (LoRaWAN^{™}), the 2G network, the 3G network, the 4G network and/or any other mobile network.

3. Modular measuring device (1) according to any one of the preceding claims, wherein the cylindrical module (11, 12, 13, 14, 15) comprises a module transmitter, a module transceiver, and/or a module receiver, which module transmitter, module transceiver, and/or module receiver are in data communication with the housing (10), preferably wherein the housing (10) and the cylindrical module (11, 12, 13, 14, 15) comprise position determining means,
and/or wherein the housing (10) comprises a first side (20) and a second side (21) opposite to the first side, and wherein the cylindrical module has opposite ends (40, 41), wherein the cylindrical module is detachably secured to the first side of the housing at one of the opposite ends thereof,
preferably wherein an additional cylindrical module (12, 13, 14, 15) is provided between the housing (10) and the cylindrical module (11, 12, 13, 14, 15), preferably wherein the additional cylindrical module is a spacing cylindrical module.

4. Modular measuring device (1) according to any one of the preceding claims, further comprising one or more additional cylindrical modules (12, 13, 14, 15) with opposite ends (40, 41), which are in data communication with the cylindrical module (11), another additional cylindrical module (12, 13, 14, 15) and/or the housing (10), optionally, wherein the one or more additional cylindrical modules (12, 13, 14, 15) comprise position determining means for determining the position of the respective additional cylindrical module,
preferably wherein the one or more additional cylindrical modules (12, 13, 14, 15) are detachably secured in series to the cylindrical module (11), to another one of the one or more additional cylindrical modules (12, 13, 14, 15), and/or, when the housing (10) comprises a first side (20) and a second side (21) opposite to the first side, to the second side of the housing at one of the opposite ends thereof, preferably wherein each of the one or more additional cylindrical modules (12, 13, 14, 15) is a spacing cylindrical module and/or is provided with an additional sensor configured for measuring a parameter of the medium external to, surrounding and/or inside the respective cylindrical module, or for measuring a parameter of the modular measuring device itself.

5. Modular measuring device (1) according to claim 4, wherein the housing (10), the cylindrical module (11) and/or the one or more additional cylindrical modules (12, 13, 14, 15) comprises a memory containing identification data related to the housing or the respective cylindrical module, which memory is in data communication with the housing (10), preferably wherein the identification data relates to whether the additional cylindrical module (12, 13, 14, 15) is a spacing module or a cylindrical module with a sensor, to the parameter measured by the sensor, the sensor, the position determining means and/or to the length of the respective cylindrical module,
preferably wherein the modular measuring device (1) is configured to transmit the identification data of each of the housing (10), the cylindrical module (11) and/or the one or more additional cylindrical modules (12, 13, 14, 15) to the external processing device (401), preferably upon a request thereto.

6. Modular measuring device (1) according to claim 4, wherein the modular measuring device is configured to transmit the identification data of the housing (10), the cylindrical module (11) and/or the one or more additional cylindrical modules (12, 13, 14, 15) in a transmitting order, and the cylindrical module and the one or more additional cylindrical modules are arranged in a module order, wherein the transmitting order corresponds to or is inverse to the module order.

7. Modular measuring device (1) according to any one of the claims 4-6, wherein each of the cylindrical module (11) and the one or more additional cylindrical modules (12, 13, 14, 15) has a first coupling part (240, 340) at one of the opposite ends and a second coupling part (244, 344) at the other of the opposite ends, preferably wherein the first coupling part of each of the cylindrical module (11) and the one or more additional cylindrical modules (12, 13, 14, 15) can be secured with the second coupling part of an adjacent cylindrical module, or vice versa,
and/or wherein each of the cylindrical module (11) and the one or more additional cylindrical modules (12, 13, 14, 15) has a first connector (260, 360) at one of the opposite ends, a second connector (379) at the other of the opposite ends, and one or more conductors (261, 262, 263, 264, 375, 376, 377, 378) for conducting energy and/or data between the first connector and the second connector, or vice versa.

8. Modular measuring device (1) according to claim 7, wherein, for each of the cylindrical module (11) and the one or more additional cylindrical modules (12, 13, 14, 15), the first connector is provided at or near the first coupling part and the second connector is provided at or near the second coupling part.

9. Modular measuring device (1) according to any one of the preceding claims, wherein the position determining means is selected from a group comprising a Global Positioning System (GPS) receiver and a Global Navigation Satellite System (GNSS) receiver,
and/or wherein an additional sensor is provided at least partially within or connected to the housing (10) and/or the cylindrical module (12),
and/or wherein the sensor (247, 347) or the additional sensor is selected from a group comprising a humidity sensor, a flow sensor, a pH sensor, a temperature sensor and an (in)clinometer,
and/or further comprising a storage medium arranged within the housing (10), wherein the storage medium is configured for storing the one or more measured parameters and the determined position at least temporarily,
and/or wherein the modular measuring device, in particular the housing, comprises a connector configured for connecting to an external device (401), wherein the connector is in data communication with the housing (10) and/or the cylindrical module (11).

10. Modular measuring device (1) according to any one of the preceding claims, further comprising signaling means arranged at least partially within the housing (10), wherein the signaling means are configured for emitting a signal, wherein the emitted signal is at least one of an alarm signal, a recognition signal and a signaling signal,
preferably wherein the signaling means comprise at least one of a Bluetooth transmitter, a light source, such as an infrared light or an LED, and a sound source for emitting the signal.

11. Processing system (400) for processing one or more measured parameters and/or one or more determined positions received from one or more modular measuring devices (1), the processing system comprising one or more modular measuring devices according to any one of the preceding claims, the processing system further comprising:
a processing device (401) in data communication with the one or more modular measuring devices for receiving one or more measured parameters and/or one or more determined positions from the one or more modular measuring devices (1), wherein the processing device is configured for processing the one or more measured parameters and the one or more determined positions received from each of the one or more modular measuring devices.

12. Processing system (400) according to claim 11, further comprising a data storage configured for storing the one or more measured parameters, one or more determined positions, and/or any data received from and/or sent to the one or more modular measuring devices,
and/or wherein the processing device (401) is configured to transmit a command to at least one of the one or more modular measuring devices (1) in order to measure the one or more parameters and/or to determine the positions of the one or more modular measuring devices,
and/or wherein, during use, the processing device (401) receives a plurality of determined positions from each of the one or more modular measuring devices (1), wherein the processing device (401) is configured, for each of the one or more modular measuring devices (1), to determine an actual position based on the plurality of determined positions.

13. Processing system (400) according to any one of the claims 11-12, wherein the one or more modular measuring devices (1) are modular measuring devices according to claim 3, wherein the processing device (401) is configured to transmit an identification command to at least one of the one or more modular measuring devices, to receive identification data of the housing, the cylindrical module (11) and/or the one or more additional cylindrical modules (12, 13, 14, 15) from at least one of the one or more modular measuring devices (1), and to determine the module order in which the cylindrical module and the one or more additional cylindrical modules are arranged for each of the one or more modular measuring devices,
and/or comprising a forecasting, nowcasting and/or backcasting device configured for forecasting, nowcasting and/or backcasting one or more parameters of the medium surrounding at least part of one of the one or more modular measuring devices, preferably wherein the forecasting, nowcasting and/or backcasting device uses at least one computer model for forecasting, nowcasting and/or backcasting the one or more parameters and/or other data, preferably wherein the processing system (400) is configured to adjust the at least one computer model on basis of the one or more measured parameters.

14. Method for processing one or more measured parameters and/or one or more determined positions received from one or more modular measuring devices according to claim 1, by means of a processing system according to claim 11, the method comprising the steps of:
for each of the one or more modular measuring devices (1), positioning the modular measuring device at any desirable position in which the modular measuring device is at least partially surrounded by a medium,
for each of the one or more modular measuring devices, measuring, with the use of the modular measuring device, one or more parameters of the medium external to, at least partially surrounding and/or inside the modular measuring device, and/or one or more parameters of the modular measuring device itself,
for each of the one or more modular measuring devices, determining, with the use of the modular measuring device, the position of the modular measuring device, and
for each of the one or more modular measuring devices, transmitting the measured one or more parameters and/or determined position to the processing device.

15. Method according to claim 14, further comprising the steps of:
for each of the one or more modular measuring devices, forecasting, nowcasting and/or backcasting at least one parameter of the medium surrounding and/or inside the respective modular measuring device at least partially, by using a computer model,
comparing the forecasted, nowcasted and/or backcasted parameter with a respective one of the measured parameters, and
providing feedback to and adjusting the computer model based on a difference between the forecasted, nowcasted and/or backcasted parameter and the respective one of the measured parameters,
and/or wherein the modular measuring device comprises one or more additional cylindrical modules (12, 13, 14, 15) with opposite ends, which are detachably secured in series to the cylindrical module (11), wherein each of the housing (10), the cylindrical module (11) and/or the one or more additional cylindrical modules (12, 13, 14, 15) comprises a memory containing identification data relating to the housing or the respective cylindrical module, which memory is in data communication with the housing, wherein the cylindrical module and the one or more additional cylindrical modules are arranged in a module order, and wherein the method comprises the steps of
transmitting an identification command from the processing device (401) to at least one of the one or more modular measuring devices,
at the at least one of the one or more modular measuring devices (1), transmitting, upon receipt of the identification command, the identification data of each of the housing (10), the cylindrical module (11) and/or the one or more additional cylindrical modules (12, 13, 14, 15) to the processing device, and
at the processing device (401), processing the identification data received from the at least one modular measuring device,
wherein the identification data of the housing (10), the cylindrical module (11) and/or the one or more additional cylindrical modules (12, 13, 14, 15) are transmitted in a transmitting order, wherein the transmitting order corresponds to or is inverse to the module order.

## Patentansprüche

1. Modulare Messeinrichtung (1) zum Messen wenigstens eines Parameters eines Mediums außerhalb der modularen Messeinrichtung, dieselbe wenigstens teilweise umgebend und/oder innerhalb derselben, wie beispielsweise eines atmosphärischen, klimatischen, Boden-, Grundwasser- und/oder Oberflächenwasser-Parameters, und/oder zum Messen wenigstens eines Parameters der modularen Messeinrichtung selbst, wie beispielsweise (relativer) Neigung, Position und/oder Lage, wobei die modulare Messeinrichtung Folgendes umfasst:
ein Gehäuse (10) und
ein zylindrisches Modul (11, 12, 13, 14, 15), das dafür konfiguriert ist, in wenigstens einem Medium platziert zu werden, wobei das zylindrische Modul zu dem Medium offen oder geschlossen ist und in Datenverbindung mit dem Gehäuse steht, **gekennzeichnet durch**
einen Sensor (247, 347), der wenigstens teilweise innerhalb des zylindrischen Moduls angeordnet und zum Messen wenigstens eines Parameters des Mediums außerhalb des zylindrischen Moduls, dasselbe wenigstens teilweise umgebend und/oder innerhalb desselben und/oder eines Parameters des zylindrischen Moduls selbst konfiguriert ist,
wobei die modulare Messeinrichtung ferner Positionsbestimmungsmittel umfasst, die wenigstens teilweise innerhalb des Gehäuses und/oder innerhalb des zylindrischen Moduls angeordnet und zum Bestimmen der Position der Positionsbestimmungsmittel konfiguriert sind, wobei die modulare Messeinrichtung zum Senden des wenigstens einen gemessenen Parameters und der bestimmten Position an eine externe Verarbeitungseinrichtung (401) und/oder Empfangen von Daten von derselben konfiguriert ist.

2. Modulare Messeinrichtung (1) nach Anspruch 1, wobei die Positionsbestimmungsmittel zum Bestimmen der Position periodisch und/oder nach Empfang einer Aufforderung dazu konfiguriert sind, und/oder wobei die modulare Messeinrichtung zum Bestimmen der Position, mit Hilfe der Positionsbestimmungsmittel, anschließend an die Anordnung der modularen Messeinrichtung in dem wenigstens einen Medium konfiguriert ist,
und/oder die einen Sender, einen Sender-Empfänger und/oder einen Empfänger umfasst, wenigstens teilweise innerhalb des Gehäuses (10) angeordnet in Datenverbindung mit dem zylindrischen Modul (11, 12, 13, 14, 15), dem Sensor (247, 347) und/oder den Positionsbestimmungsmitteln, wobei der Sender und/oder der Sender-Empfänger zum Senden des gemessenen Parameters und/oder der bestimmten Position an oder von der externen Verarbeitungseinrichtung (401) konfiguriert sind, vorzugsweise wobei der Sender, der Sender-Empfänger und/oder der Empfänger verdrahtet oder drahtlos mit dem zylindrischen Modul (11, 12, 13, 14, 15), dem Sensor (247, 347) und/oder den Positionsbestimmungsmitteln verbunden sind,
vorzugsweise wobei der Sender, der Sender-Empfänger und/oder der Empfänger dafür konfiguriert sind, in einem drahtgebundenen und/oder einem mobilen Netz, wie beispielsweise einem Long Range Wide Area Network (LoRaWAN^{™}), einem 2G-Netz, einem 3G-Netz, einem 4G-Netz und/oder einem beliebigen anderen mobilen Netz, zu arbeiten,
die vorzugsweise einen zusätzlichen Sender, einen zusätzlichen Sender-Empfänger und/oder einen zusätzlichen Empfänger umfasst, wenigstens teilweise innerhalb des Gehäuses (10) angeordnet in Datenverbindung mit dem zylindrischen Modul (11, 12, 13, 14, 15), dem Sensor (247, 347) und/oder den Positionsbestimmungsmitteln zum Senden des gemessenen Parameters und der bestimmten Position an und/oder von der externen Verarbeitungseinrichtung (401), vorzugsweise wobei der Sender, der Sender-Empfänger und/oder der Empfänger dafür konfiguriert sind, in einem von dem Long Range Wide Area Network (LoRaWAN^{™}), dem 2G-Netz, dem 3G-Netz, dem 4G-Netz und/oder einem beliebigen anderen mobilen Netz zu arbeiten, und der zusätzliche Sender, der zusätzliche Sender-Empfänger und/oder der zusätzliche Empfänger dafür konfiguriert sind, in einem anderen von dem Long Range Wide Area Network (LoRaWAN^{™}), dem 2G-Netz, dem 3G-Netz, dem 4G-Netz und/oder einem beliebigen anderen mobilen Netz zu arbeiten.

3. Modulare Messeinrichtung (1) nach einem der vorhergehenden Ansprüche, wobei das zylindrische Modul (11, 12, 13, 14, 15) einen Modulsender, einen Modul-Sender-Empfänger und/oder einen Modulempfänger umfasst, wobei der Modulsender, der Modul-Sender-Empfänger und/oder der Modulempfänger in Datenverbindung mit dem Gehäuse (10) stehen, vorzugsweise wobei das Gehäuse (10) und das zylindrische Modul (11, 12, 13, 14, 15) Positionsbestimmungsmittel umfassen,
und/oder wobei das Gehäuse (10) eine erste Seite (20) und eine zweite Seite (21), entgegengesetzt zu der ersten Seite, umfasst und wobei das zylindrische Modul entgegengesetzte Enden (40, 41) aufweist, wobei das zylindrische Modul an einem der entgegengesetzten Enden desselben abnehmbar an der ersten Seite des Gehäuses befestigt ist,
vorzugsweise wobei ein zusätzliches zylindrisches Modul (12, 13, 14, 15) zwischen dem Gehäuse (10) und dem zylindrischen Modul (11, 12, 13, 14, 15) bereitgestellt wird, vorzugsweise wobei das zusätzliche zylindrische Modul ein zylindrisches Abstandshaltermodul ist.

4. Modulare Messeinrichtung (1) nach einem der vorhergehenden Ansprüche, die ferner ein oder mehrere zusätzliche zylindrische Module (12, 13, 14, 15) mit entgegengesetzten Enden (40, 41) umfasst, die in Datenverbindung mit dem zylindrischen Modul (11), einem anderen zusätzlichen zylindrischen Modul (12, 13, 14, 15) und/oder dem Gehäuse (10) stehen, wahlweise wobei das eine oder die mehreren zusätzlichen zylindrischen Module (12, 13, 14, 15) Positionsbestimmungsmodule zum Bestimmen der Position des jeweiligen zusätzlichen zylindrischen Moduls umfassen,
vorzugsweise wobei das eine oder die mehreren zusätzlichen zylindrischen Module (12, 13, 14, 15) abnehmbar in Reihe an dem zylindrischen Modul (11), an einem anderen von dem einen oder den mehreren zusätzlichen zylindrischen Modulen (12, 13, 14, 15) und/oder, wenn das Gehäuse (10) eine erste Seite (20) und eine zweite Seite (21), entgegengesetzt zu der ersten Seite, umfasst, an der zweiten Seite des Gehäuses an einem der entgegengesetzten Enden desselben befestigt sind, vorzugsweise wobei jedes von dem einen oder den mehreren zusätzlichen zylindrischen Module (12, 13, 14, 15) ein zylindrisches Abstandshaltermodul ist und/oder mit einem zusätzlichen Sensor versehen ist, der zum Messen wenigstens eines Parameters des Mediums außerhalb des jeweiligen zylindrischen Moduls, dasselbe umgebend und/oder innerhalb desselben oder zum Messen eines Parameters der modularen Messeinrichtung selbst konfiguriert ist.

5. Modulare Messeinrichtung (1) nach Anspruch 4, wobei das Gehäuse (10), das zylindrische Modul (11) und/oder das eine oder die mehreren zusätzlichen zylindrischen Module (12, 13, 14, 15) einen Speicher umfassen, der Identifikationsdaten enthält, die mit dem Gehäuse oder dem jeweiligen zylindrischen Modul in Beziehung stehen, wobei der Speicher in Datenverbindung mit dem Gehäuse (10) steht, vorzugsweise wobei sich die Identifikationsdaten darauf, ob das zusätzliche zylindrische Modul (12, 13, 14, 15) ein Abstandshaltermodul oder ein zylindrisches Modul mit einem Sensor ist, auf den durch den Sensor gemessenen Parameter, den Sensor, die Positionsbestimmungsmittel und/oder auf die Länge des jeweiligen zylindrischen Moduls beziehen,
vorzugsweise wobei die modulare Messeinrichtung (1) dafür konfiguriert ist, die Identifikationsdaten jedes von dem Gehäuse (10), dem zylindrischen Modul (11) und/oder dem einen oder den mehreren zusätzlichen zylindrischen Modulen (12, 13, 14, 15) an die externe Verarbeitungseinrichtung (401) zu senden, vorzugsweise auf eine Aufforderung dazu hin.

6. Modulare Messeinrichtung (1) nach Anspruch 4, wobei die modulare Messeinrichtung dafür konfiguriert ist, die Identifikationsdaten des Gehäuses (10), des zylindrischen Moduls (11) und/oder des einen oder der mehreren zusätzlichen zylindrischen Module (12, 13, 14, 15) in einer Sendereihenfolge zu senden, und das zylindrische Modul und/oder das eine oder die mehreren zusätzlichen zylindrischen Module in einer Modulreihenfolge angeordnet sind, wobei die Sendereihenfolge der Modulreihenfolge entspricht oder umgekehrt zu derselben ist.

7. Modulare Messeinrichtung (1) nach einem der Ansprüche 4 bis 6, wobei jedes von dem zylindrischen Modul (11) und dem einen oder den mehreren zusätzlichen zylindrischen Modulen (12, 13, 14, 15) einen ersten Kupplungsteil (240, 340) an einem der entgegengesetzten Enden und einen zweiten Kupplungsteil (244, 344) an dem anderen der entgegengesetzten Enden aufweist, vorzugsweise wobei der erste Kupplungsteil jedes von dem zylindrischen Modul (11) und dem einen oder den mehreren zusätzlichen zylindrischen Module (12, 13, 14, 15) mit dem zweiten Kupplungsteil eines benachbarten zylindrischen Moduls oder umgekehrt befestigt werden kann,
und/oder wobei jedes von dem zylindrischen Modul (11) und dem einen oder den mehreren zusätzlichen zylindrischen Modulen (12, 13, 14, 15) einen ersten Verbinder (260, 360) an einem der entgegengesetzten Enden, einen zweiten Verbinder (379) an dem anderen der entgegengesetzten Enden und einen oder mehrere Leiter (261, 262, 263, 264, 375, 376, 377, 378) zum Leiten von Energie und/oder Daten zwischen dem ersten Verbinder und dem zweiten Verbinder oder umgekehrt aufweist.

8. Modulare Messeinrichtung (1) nach Anspruch 7, wobei, für jedes von dem zylindrischen Modul (11) und dem einen oder den mehreren zusätzlichen zylindrischen Modulen (12, 13, 14, 15), der erste Verbinder an oder nahe dem ersten Kupplungsteil bereitgestellt wird und der zweite Verbinder an oder nahe dem zweiten Kupplungsteil bereitgestellt wird.

9. Modulare Messeinrichtung (1) nach einem der vorhergehenden Ansprüche, wobei das Positionsbestimmungsmittel ausgewählt ist aus einer Gruppe, die einen Global-Positioning-System- (GPS-) Empfänger und einem Global-Navigation-Satellite-System- (GNSS-) Empfänger umfasst,
und/oder wobei ein zusätzlicher Sensor wenigstens teilweise innerhalb des Gehäuses (10) und/oder des zylindrischen Moduls (12) bereitgestellt wird, und/oder wobei der Sensor (247, 347) oder der zusätzliche Sensor aus einer Gruppe ausgewählt ist, die einen Feuchtigkeitssensor, einen Strömungssensor, einen pH-Sensor, einen Temperatursensor und ein (In) Klinometer umfasst,
und/oder die ferner ein Speichermedium umfasst, das innerhalb des Gehäuses (10) angeordnet ist, wobei das Speichermedium zum wenigstens zeitweiligen Speichern des einen oder der mehreren gemessenen Parameter und der bestimmten Position konfiguriert ist,
und/oder wobei die modulare Messeinrichtung, insbesondere das Gehäuse, einen Verbinder umfasst, der zum Verbinden mit einer externen Einrichtung (401) konfiguriert ist, wobei der Verbinder in Datenverbindung mit dem Gehäuse (10) und/oder dem zylindrischen Modul (11) steht.

10. Modulare Messeinrichtung (1) nach einem der vorhergehenden Ansprüche, die ferner Signalisierungsmittel umfasst, die wenigstens teilweise innerhalb des Gehäuses (10) angeordnet sind, wobei die Signalisierungsmittel zum Emittieren eines Signals konfiguriert sind, wobei das emittierte Signal wenigstens eines von einem Alarmsignal, einem Erkennungssignal und einem Signalisierungssignal ist,
vorzugsweise wobei die Signalisierungsmittel wenigstens eines von einem Bluetooth-Sender, einer Lichtquelle, wie beispielsweise einer Infrarotleuchte oder einer LED, und einer Schallquelle zum Emittieren des Signals umfassen.

11. Verarbeitungssystem (400) zum Verarbeiten eines oder mehrerer gemessener Parameter und/oder einer oder mehrerer bestimmter Positionen, die von einer oder mehreren modularen Messeinrichtungen (1) empfangen werden, wobei das Verarbeitungssystem eine oder mehrere modularen Messeinrichtungen nach einem der vorhergehenden Ansprüche umfasst, wobei das Verarbeitungssystem ferner Folgendes umfasst:
eine Verarbeitungseinrichtung (401) in Datenverbindung mit der einen oder den mehreren modularen Messeinrichtungen zum Empfangen eines oder mehrerer gemessener Parameter und/oder einer oder mehrerer bestimmter Positionen von der einen oder den mehreren modularen Messeinrichtungen (1), wobei die Verarbeitungseinrichtung zum Verarbeiten des einen oder der mehreren gemessenen Parameter und der einen oder der mehreren bestimmten Positionen, die von jeder der einen oder den mehreren modularen Messeinrichtungen empfangen werden, konfiguriert ist.

12. Verarbeitungssystem (400) nach Anspruch 11, die ferner einen Datenspeicher umfasst, der zum Speichern des einen oder der mehreren gemessenen Parameter, der einen oder der mehreren bestimmten Positionen und/oder jeglicher Daten, die von jeder der einen oder den mehreren modularen Messeinrichtungen empfangen und/oder an dieselben gesendet werden, konfiguriert ist,
und/oder wobei die Verarbeitungseinrichtung (401) dafür konfiguriert ist, einen Befehl an wenigstens eine von der einen oder den mehreren modularen Messeinrichtungen (1) zu senden, um den einen oder die mehreren Parameter zu messen und/oder die Positionen der einen oder der mehreren modularen Messeinrichtungen zu bestimmen,
und/oder wobei, während der Anwendung, die Verarbeitungseinrichtung (401) eine Vielzahl von bestimmten Positionen von der einen oder den mehreren modularen Messeinrichtungen (1) empfängt, wobei die Verarbeitungseinrichtung (401) dafür konfiguriert ist, für jede von der einen oder den mehreren modularen Messeinrichtungen (1), auf Grundlage der Vielzahl bestimmter Positionen eine tatsächliche Position zu bestimmen.

13. Verarbeitungssystem (400) nach einem der Ansprüche 11 bis 12, wobei die eine oder die mehreren modularen Messeinrichtungen (1) modulare Messeinrichtungen nach Anspruch 3 sind, wobei die Verarbeitungseinrichtung (401) dafür konfiguriert ist, einen Identifikationsbefehl an wenigstens eine von der einen oder den mehreren modularen Messeinrichtungen zu senden, Identifikationsdaten des Gehäuses, des zylindrischen Moduls (11) und/oder des einen oder der mehreren zusätzlichen zylindrischen Module (12, 13, 14, 15) von wenigstens einer der einen oder der mehreren modularen Messeinrichtungen (1) zu empfangen und die Modulreihenfolge, in der das zylindrische Modul und das eine oder die mehreren zusätzlichen zylindrischen Module angeordnet sind, für jede von der einen oder den mehreren modularen Messeinrichtungen zu bestimmen,
und/oder das eine Vorhersage-, Nowcasting- und/oder Backcasting-Einrichtung umfasst, die zum Vorhersagen, Nowcasten und/oder Backcasten eines oder mehrerer Parameter des Mediums, das wenigstens einen Teil einer von der einen oder den mehreren modularen Messeinrichtungen umgibt, konfiguriert ist, vorzugsweise wobei die Vorhersage-, Nowcasting- und/oder Backcasting-Einrichtung wenigstens ein Rechnermodell zum Vorhersagen, Nowcasten und/oder Backcasten des einen oder der mehreren Parameter und/oder anderer Daten verwendet, vorzugsweise wobei das System (400) dafür konfiguriert ist, das wenigstens eine Rechnermodell auf Grundlage des einen oder der mehreren gemessenen Parameter anzupassen.

14. Verfahren zum Verarbeiten eines oder mehrerer gemessener Parameter und/oder einer oder mehrerer bestimmter Positionen, die von einer oder mehreren modularen Messeinrichtungen nach Anspruch 1 empfangen werden, mit Hilfe eines Verarbeitungssystems nach Anspruch 11, wobei das Verfahren die folgenden Schritte umfasst:
für jede von der einen oder den mehreren modularen Messeinrichtungen (1), Positionieren der modularen Messeinrichtung an einer beliebigen wünschenswerten Position, in der die modulare Messeinrichtung wenigstens teilweise von einem Medium umgeben ist,
für jede von der einen oder den mehreren modularen Messeinrichtungen, Messen, mit der Verwendung der modularen Messeinrichtung, eines oder mehrerer Parameter des Mediums außerhalb der modularen Messeinrichtung, dieselbe wenigstens teilweise umgebend und/oder innerhalb derselben und eines oder mehrerer Parameter der modularen Messeinrichtung selbst,
für jede von der einen oder den mehreren modularen Messeinrichtungen, Bestimmen, mit der Verwendung der modularen Messeinrichtung, der Position der modularen Messeinrichtung und
für jede von der einen oder den mehreren modularen Messeinrichtungen, Senden des/der gemessenen einen oder mehreren Parameter und/oder der gemessenen Position an die Verarbeitungseinrichtung.

15. Verfahren nach Anspruch 14, das ferner die folgenden Schritte umfasst:
für jede von der einen oder den mehreren modularen Messeinrichtungen, Vorhersagen, Nowcasten und/oder Backcasten wenigstens eines Parameters des Mediums, wenigstens teilweise die jeweilige modulare Messeinrichtung umgebend und/oder innerhalb derselben, durch Verwenden eines Rechnermodells,
Vergleichen des vorhergesagten, nowcasteten und/oder backcasteten Parameters mit einem jeweiligen der gemessenen Parameter und
Bereitstellen einer Rückmeldung an das Rechnermodell und Anpassen desselben auf Grundlage eines Unterschieds zwischen dem vorhergesagten, nowcasteten und/oder backcasteten Parameter und dem jeweiligen der gemessenen Parameter,
und/oder wobei die modulare Messeinrichtung ein oder mehrere zusätzliche zylindrische Module (12, 13, 14, 15) mit entgegengesetzten Enden umfasst, die abnehmbar in Reihe an dem zylindrischen Modul (11) befestigt sind, wobei jedes von dem Gehäuse (10), dem zylindrischen Modul (11) und/oder dem einen oder den mehreren zusätzlichen zylindrischen Modulen (12, 13, 14, 15) einen Speicher umfasst, der Identifikationsdaten enthält, die mit dem Gehäuse oder dem jeweiligen zylindrischen Modul in Beziehung stehen, wobei der Speicher in Datenverbindung mit dem Gehäuse steht, wobei das zylindrische Modul und das eine oder die mehreren zusätzlichen zylindrischen Module in einer Modulreihenfolge angeordnet sind und wobei das Verfahren die folgenden Schritte umfasst:
Senden eines Identifikationsbefehls von der Verarbeitungseinrichtung (401) an wenigstens eine von der einen oder den mehreren modularen Messeinrichtungen,
an der wenigstens einen von der einen oder den mehreren modularen Messeinrichtungen (1), Senden, nach Empfang des Identifikationsbefehls, der Identifikationsdaten jedes von dem Gehäuse (10), dem zylindrischen Modul (11) und/oder dem einen oder den mehreren zusätzlichen zylindrischen Modulen (12, 13, 14, 15) an die Verarbeitungseinrichtung und
an der Verarbeitungseinrichtung (401), Verarbeiten der Identifikationsdaten, die von der wenigstens einen modularen Messeinrichtung empfangen werden,
wobei die Identifikationsdaten des Gehäuses (10), des zylindrischen Moduls (11) und/oder des einen oder der mehreren zusätzlichen zylindrischen Module (12, 13, 14, 15) in einer Sendereihenfolge gesendet werden, wobei die Sendereihenfolge der Modulreihenfolge entspricht oder umgekehrt zu derselben ist.

## Revendications

1. Dispositif de mesure modulaire (1) destiné à mesurer au moins un paramètre d'un milieu extérieur à, entourant au moins partiellement et/ou à l'intérieur du dispositif de mesure modulaire, tel qu'un paramètre atmosphérique, climatique, de sol, d'eau souterraine et/ou d'eau de surface, et/ou destiné à mesurer au moins un paramètre du dispositif de mesure modulaire lui-même, tel que une inclinaison, une position et/ou un emplacement (relatifs), le dispositif de mesure modulaire comprenant :
un boîtier (10) ; et
un module cylindrique (11, 12, 13, 14, 15) configuré pour être placé dans au moins un milieu, dans lequel le module cylindrique est ouvert ou fermé vis-à-vis du milieu et est en communication de données avec le boîtier ; **caractérisé par**
un capteur (247, 347) agencé au moins partiellement dans le module cylindrique et configuré pour mesurer au moins un paramètre du milieu extérieur à, entourant au moins partiellement et/ou à l'intérieur du module cylindrique, et/ou un paramètre du dispositif de mesure modulaire lui-même,
le dispositif de mesure modulaire comprenant en outre un moyen de détermination de position agencé au moins partiellement dans le boîtier et/ou dans le module cylindrique, et configuré pour déterminer la position du moyen de détermination de position, dans lequel le dispositif de mesure modulaire est configuré pour transmettre le au moins un paramètre mesuré et la position déterminée au et/ou recevoir des données en provenance du dispositif de traitement externe (401).

2. Dispositif de mesure modulaire (1) selon la revendication 1, dans lequel le moyen de détermination de position est configuré pour déterminer la position de manière périodique et/ou à réception d'une requête en ce sens,
et/ou dans lequel le dispositif de mesure modulaire est configuré pour déterminer la position, à l'aide du moyen de détermination de positon, à la suite de l'agencement du dispositif de mesure modulaire dans le au moins un milieu,
et/ou comprenant un émetteur, un émetteur-récepteur et/ou un récepteur agencés au moins partiellement dans le boîtier (10) en communication de données avec le module cylindrique (11, 12, 13, 14, 15), le capteur (247, 347) et/ou le moyen de détermination de position, dans lequel l'émetteur et/ou l'émetteur-récepteur sont configurés pour transmettre le paramètre mesuré et/ou la position déterminée à ou depuis le dispositif de traitement externe (401), de préférence dans lequel l'émetteur, l'émetteur-récepteur et/ou le récepteur sont câblés ou connectés sans fil au module cylindrique (11, 12, 13, 14, 15), au capteur (247, 347) et/ou au moyen de détermination de position,
de préférence dans lequel l'émetteur, l'émetteur-récepteur et/ou le récepteur sont configurés pour fonctionner dans un réseau câblé et/ou mobile, tel qu'un réseau étendu longue-portée (LoRaWAN^{™}), un réseau 2G, un réseau 3G, un réseau 4G et/ou tout autre réseau mobile,
de préférence comprenant un émetteur supplémentaire, un émetteur-récepteur supplémentaire et/ou un récepteur supplémentaire agencés au moins partiellement dans le boîtier (10) et en communication de données avec le module cylindrique (11, 12, 13, 14, 15), le capteur (247, 347) et/ou le moyen de détermination de position pour transmettre le paramètre mesuré et la position déterminée au et/ou depuis le dispositif de traitement externe (401), de préférence dans lequel l'émetteur, l'émetteur-récepteur et/ou le récepteur sont configurés pour fonctionner dans l'un parmi le réseau étendu longue-portée (LoRaWAN^{™}), le réseau 2G, le réseau 3G, le réseau 4G et/ou tout autre réseau mobile, et l'émetteur supplémentaire, l'émetteur-récepteur supplémentaire et/ou le récepteur supplémentaire sont configurés pour fonctionner dans un autre parmi le réseau étendu longue-portée (LoRaWAN^{™}), le réseau 2G, le réseau 3G, le réseau 4G et/ou tout autre réseau mobile.

3. Dispositif de mesure modulaire (1) selon l'une quelconque des revendications précédentes, dans lequel le module cylindrique (11, 12, 13, 14, 15) comprend un émetteur de module, un émetteur-récepteur de module et/ou un récepteur de module, lesquels émetteur de module, émetteur-récepteur de module et/ou récepteur de module étant en communication de données avec le boîtier (10), de préférence dans lequel le boîtier (10) et le module cylindrique (11, 12, 13, 14, 15) comprennent un moyen de détermination de position,
et/ou dans lequel le boîtier (10) comprend un premier côté (20) et un second côté (21) opposé au premier côté, et dans lequel le module cylindrique présente des extrémités opposées (40, 41), dans lequel le module cylindrique est fixé de manière détachable au premier côté du boîtier au niveau de l'une des extrémités opposées de celui-ci,
de préférence dans lequel un module cylindrique supplémentaire (12, 13, 14, 15) est disposé entre le boîtier (10) et le module cylindrique (11, 12, 13, 14, 15), de préférence dans lequel le module cylindrique supplémentaire est un module cylindrique d'espacement.

4. Dispositif de mesure modulaire (1) selon l'une quelconque des revendications précédentes, comprenant en outre un ou plusieurs modules cylindriques supplémentaires (12, 13, 14, 15) pourvus d'extrémités opposées (40, 41), qui sont en communication de données avec le module cylindrique (11), un autre module cylindrique supplémentaire (12, 13, 14, 15) et/ou le boîtier (10), optionnellement, dans lequel les un ou plusieurs modules cylindriques supplémentaires (12, 13, 14, 15) comprennent un moyen de détermination de position destiné à déterminer la position du module cylindrique supplémentaire respectif,
de préférence dans lequel les un ou plusieurs modules cylindriques supplémentaires (12, 13, 14, 15) sont fixés de manière détachable en série au module cylindrique (11), à un autre des un ou plusieurs modules cylindriques supplémentaires (12, 13, 14, 15), et/ou, lorsque le boîtier (10) comprend un premier côté (20) et un second côté (21) opposé au premier côté, au second côté du boîtier au niveau d'une des extrémités opposées de celui-ci, de préférence dans lequel chacun des un ou plusieurs modules cylindriques supplémentaires (12, 13, 14, 15) est un module cylindrique d'espacement et/ou est pourvu d'un capteur supplémentaire configuré pour mesurer un paramètre du milieu extérieur à, entourant et/ou à l'intérieur du module cylindrique respectif, ou pour mesurer un paramètre du dispositif de mesure modulaire lui-même.

5. Dispositif de mesure modulaire (1) selon la revendication 4, dans lequel le boîtier (10), le module cylindrique (11) et/ou les un ou plusieurs modules cylindriques supplémentaires (12, 13, 14, 15) comprennent une mémoire contenant des données d'identification liées au boîtier ou au module cylindrique respectif, laquelle mémoire est en communication de données avec le boîtier (10), de préférence dans lequel les données d'identification se rapportent au fait de savoir si le module cylindrique supplémentaire (12, 13, 14, 15) est un module d'espacement ou un module cylindrique pourvu d'un capteur, au paramètre mesuré par le capteur, au capteur, au moyen de détermination de position et/ou à la longueur du module cylindrique respectif,
de préférence dans lequel le dispositif de mesure modulaire (1) est configuré pour transmettre les données d'identification de chacun parmi le boîtier (10), le module cylindrique (11) et/ou les un ou plusieurs modules cylindriques supplémentaires (12, 13, 14, 15) au dispositif de traitement externe (401), de préférence lors d'une requête en ce sens.

6. Dispositif de mesure modulaire (1) selon la revendication 4, dans lequel le dispositif de mesure modulaire est configuré pour transmettre les données d'identification du boîtier (10), du module cylindrique (11) et/ou des un ou plusieurs modules cylindriques supplémentaires (12, 13, 14, 15) dans un ordre de transmission, et le module cylindrique et les un ou plusieurs modules cylindriques supplémentaires sont agencés dans un ordre de modules, dans lequel l'ordre de transmission correspond à ou est contraire à l'ordre de modules.

7. Dispositif de mesure modulaire (1) selon l'une quelconque des revendications 4-6, dans lequel chacun parmi le module cylindrique et les un ou plusieurs modules cylindriques supplémentaires (12, 13, 14, 15) présente une première partie d'accouplement (240, 340) au niveau d'une des extrémités opposées et une seconde partie d'accouplement (244, 344) au niveau de l'autre des extrémités opposées, de préférence dans lequel la première partie d'accouplement de chacun parmi le module cylindrique (11) et les un ou plusieurs modules cylindriques supplémentaires (12, 13, 14, 15) peut être fixée à la seconde partie d'accouplement d'un module cylindrique adjacent, ou vice versa,
et/ou dans lequel chacun parmi le module cylindrique et les un ou plusieurs modules cylindriques supplémentaires (12, 13, 14, 15) présente un premier connecteur (260, 360) au niveau d'une des extrémités opposées, un second connecteur (379) au niveau de l'autre des extrémités opposées, et un ou plusieurs conducteurs (261, 262, 263, 264, 375, 376, 377, 378) pour conduire de l'énergie et/ou des données entre le premier connecteur et le second connecteur, ou vice versa.

8. Dispositif de mesure modulaire (1) selon la revendication 7, dans lequel pour chacun parmi le module cylindrique et les un ou plusieurs modules cylindriques supplémentaires (12, 13, 14, 15), le premier connecteur est disposé au niveau ou près de la première partie d'accouplement et le second connecteur est disposé au niveau ou près de la seconde partie d'accouplement.

9. Dispositif de mesure modulaire (1) selon l'une quelconque des revendications précédentes, dans lequel le moyen de détermination de position est sélectionné à partir d'un groupe comprenant un récepteur de système de localisation mondial (GPS) et un récepteur de système mondial de navigation par satellite (GNSS),
et/ou dans lequel un capteur supplémentaire est disposé au moins partiellement dans ou connecté au boîtier (10) et/ou au module cylindrique (12),
et/ou dans lequel le capteur (347, 347) ou le capteur supplémentaire est sélectionné à partir d'un groupe comprenant un capteur d'humidité, un capteur d'écoulement, un capteur de pH, un capteur de température et un (in)clinomètre,
et/ou comprenant en outre un support de stockage agencé dans le boîtier (10), dans lequel le support de stockage est configuré pour stocker les un ou plusieurs paramètres mesurés et la position déterminée au moins provisoirement,
et/ou dans lequel le dispositif de mesure modulaire, en particulier le boîtier, comprend un connecteur configuré pour connexion à un dispositif externe (401), dans lequel le connecteur est en communication de données avec le boîtier (10) et/ou le module cylindrique (11).

10. Dispositif de mesure modulaire (1) selon l'une quelconque des revendications précédentes, comprenant en outre un moyen de signalisation agencé au moins partiellement dans le boîtier (10), dans lequel le moyen de signalisation est configuré pour émettre un signal, dans lequel le signal émis est au moins un parmi un signal d'alarme, un signal de reconnaissance et un signal de signalisation,
de préférence dans lequel le moyen de signalisation comprend au moins un parmi un émetteur Bluetooth, une source de lumière, telle qu'une lumière infrarouge ou une LED, et une source sonore pour émettre le signal.

11. Système de traitement (400) destiné à traiter un ou plusieurs paramètres mesurés et/ou une ou plusieurs positions déterminées reçus en provenance d'un ou plusieurs dispositifs de mesure modulaires (1), le système de traitement comprenant un ou plusieurs dispositifs de mesure modulaires selon l'une quelconque des revendications précédentes, le système de traitement comprenant en outre :
un dispositif de traitement (401) en communication de données avec les un ou plusieurs dispositifs de mesure modulaires pour recevoir un ou plusieurs paramètres mesurés et/ou une ou plusieurs positions déterminées en provenance des un ou plusieurs dispositifs de mesure modulaires (1), dans lequel le dispositif de traitement est configuré pour traiter les un ou plusieurs paramètres mesurés et les une ou plusieurs positions déterminées reçus en provenance de chacun des un ou plusieurs dispositifs de mesure modulaires.

12. Système de traitement (400) selon la revendication 11, comprenant en outre un stockage de données configuré pour stocker les un ou plusieurs paramètres mesurés, les une ou plusieurs positions déterminées et/ou toute donnée reçue en provenance de et/ou envoyée aux un ou plusieurs dispositifs de mesure modulaires,
et/ou dans lequel le dispositif de traitement (401) est configuré pour transmettre une instruction au moins un des un ou plusieurs dispositifs de mesure modulaires (1) afin de mesurer les un ou plusieurs paramètres et/ou de déterminer les positions des un ou plusieurs dispositifs de mesure modulaires,
et/ou dans lequel, durant l'utilisation, le dispositif de traitement (401) reçoit une pluralité de positions déterminées provenant de chacun des un ou plusieurs dispositifs de mesure modulaires (1), dans lequel le dispositif de traitement (401) est configuré, pour chacun des un ou plusieurs dispositifs de mesure modulaires (1), pour déterminer une position réelle sur la base de la pluralité de positions déterminées.

13. Système de traitement (400) selon l'une quelconque des revendications 11-12, dans lequel les un ou plusieurs dispositifs de mesure modulaires (1) sont des dispositifs de mesure modulaires selon la revendication 3, dans lequel le dispositif de traitement (401) est configuré pour transmettre une instruction d'identification à au moins l'un des un ou plusieurs dispositifs de mesure modulaires, pour recevoir des données d'identification du boîtier, du module cylindrique (11) et/ou des un ou plusieurs modules cylindriques supplémentaires (12, 13, 14, 15) en provenance d'au moins un des un ou plusieurs dispositifs de mesure modulaires (1), et pour déterminer l'ordre de modules dans lequel le module cylindrique et les un ou plusieurs dispositifs de mesure modulaires sont agencés pour chacun des un ou plusieurs dispositifs de mesure modulaires,
et/ou comprenant un dispositif de prévision, de prévision immédiate et/ou de prévision à rebours configuré pour la prévision, la prévision immédiate et/ou la prévision à rebours d'un ou plusieurs paramètres du milieu entourant au moins une partie d'un des un ou plusieurs dispositifs de mesure modulaires, de préférence dans lequel le dispositif de prévision, de prévision immédiate et/ou de prévision à rebours utilise au moins un modèle informatique pour la prévision, la prévision immédiate et/ou la prévision à rebours des un ou plusieurs paramètres et/ou d'autres données, de préférence dans lequel le système de traitement (400) est configuré pour ajuster le au moins un modèle informatique sur la base des un ou plusieurs paramètres mesurés.

14. Procédé de traitement d'un ou plusieurs paramètres mesurés et/ou d'une ou plusieurs positions déterminées reçus en provenance d'un ou plusieurs dispositifs de mesure modulaires selon la revendication 1, à l'aide d'un système de traitement selon la revendication 11, le procédé comprenant les étapes consistant à :
pour chacun des un ou plusieurs dispositifs de mesure modulaires (1), positionner le dispositif de mesure modulaire à toute position souhaitable dans laquelle le dispositif de mesure modulaire est au moins partiellement entouré par un milieu,
pour chacun des un ou plusieurs dispositifs de mesure modulaires, mesurer, avec l'utilisation du dispositif de mesure modulaire, un ou plusieurs paramètres du milieu extérieur à, entourant au moins partiellement et/ou à l'intérieur du dispositif de mesure modulaire, et/ou un ou plusieurs paramètres du dispositif de mesure modulaire lui-même,
pour chacun des un ou plusieurs dispositifs de mesure modulaires, déterminer, avec l'utilisation du dispositif de mesure modulaire, la position du dispositif de mesure modulaire, et
pour chacun des un ou plusieurs dispositifs de mesure modulaires, transmettre les un ou plusieurs paramètres mesurés et/ou la position déterminée au dispositif de traitement.

15. Procédé selon la revendication 14, comprenant en outre les étapes de :
pour chacun des un ou plusieurs dispositifs de mesure modulaires, prévision, prévision immédiate et/ou prévision à rebours d'au moins un paramètre du milieu entourant et/ou à l'intérieur du dispositif de mesure modulaire respectif au moins partiellement, en utilisant un modèle informatique,
comparaison du paramètre prévu, prévu immédiatement et/ou prévu à rebours avec un des paramètres mesurés respectif, et
fourniture d'un retour au modèle informatique et ajustement de ce dernier sur la base d'une différence entre le paramètre prévu, prévu immédiatement et/ou prévu à rebours et le paramètre respectif des paramètres mesurés,
et/ou dans lequel le dispositif de mesure modulaire comprend un ou plusieurs modules cylindriques supplémentaires (12, 13, 14, 15) pourvus d'extrémités opposées, qui sont fixés de manière détachable en série au module cylindrique (11), dans lequel chacun parmi le boîtier (10), le module cylindrique (11) et/ou les un ou plusieurs modules cylindriques supplémentaires (12, 13, 14, 15) comprend un mémoire contenant des données d'identification liées au boîtier ou au module cylindrique respectif, laquelle mémoire est en communication de données avec le boîtier, dans lequel le module cylindrique et les un ou plusieurs modules cylindriques supplémentaires sont agencés dans un ordre de modules, et dans lequel le procédé comprend les étapes de
transmission d'une instruction d'identification depuis le dispositif de traitement (401) à au moins un des un ou plusieurs dispositifs de mesure modulaires,
au niveau de l'au moins un des un ou plusieurs dispositifs de mesure modulaires (1), transmission, à réception de l'instruction d'identification, des données d'identification de chacun parmi le boîtier (10), le module cylindrique (11) et/ou les un ou plusieurs modules cylindriques supplémentaires (12, 13, 14, 15) au dispositif de traitement, et
au niveau du dispositif de traitement (401), traitement des données d'identification reçues en provenance de l'au moins un dispositif de mesure modulaire,
dans lequel les données d'identification du boîtier (10), du module cylindrique (11) et/ou des un ou plusieurs modules cylindriques supplémentaires (12, 13, 14, 15) sont transmises dans un ordre de transmission, dans lequel l'ordre de transmission correspond à l'ordre de modules ou est contraire à celui-ci.
